Europäisches Patentamt

(19) European Patent Office

Office européen des brevets

(11) **EP 0 474 751 B1**

(12) **EUROPEAN PATENT SPECIFICATION**

(45) Date of publication and mention
of the grant of the patent:
**23.04.1997 Bulletin 1997/17**

(21) Application number: **90909190.2**

(22) Date of filing: **30.05.1990**

(51) Int. Cl.$^6$: **A61B 9/00**

(86) International application number:
**PCT/NL90/00079**

(87) International publication number:
**WO 90/14794 (13.12.1990 Gazette 1990/28)**

(54) **A METHOD FOR ASSAYING NEURAL SIGNALS**

VERFAHREN FÜR DIE ANALYSE VON NERVENSIGNALEN

PROCEDE POUR ANALYSER DES SIGNAUX NERVEUX

(84) Designated Contracting States:
**AT BE CH DE DK ES FR GB IT LI LU NL SE**

(30) Priority: **30.05.1989 NL 8901367**

(43) Date of publication of application:
**18.03.1992 Bulletin 1992/12**

(73) Proprietors:
• **LERNER, Inna**
**NL-1081 HE Amsterdam (NL)**
• **LERNER, Edward Naumovich**
**1083 GP Amsterdam (NL)**

(72) Inventors:
• **LERNER, Eduard, Naumovich**
**NL-1083 GP AMSTERDAM (NL)**
• **LERNER, Leonid**
**San Francisco; Cal. 94122 (US)**

(74) Representative: **de Bruijn, Leendert C. et al**
**Nederlandsch Octrooibureau**
**P.O. Box 29720**
**2502 LS Den Haag (NL)**

(56) References cited:
**US-A- 4 064 870** **US-A- 4 557 271**

• **MEDICAL AND BIOLOGICAL ENGINEERING AND COMPUTING. vol. 25, no. 5, September 1987, STEVENAGE GB pages 567 - 572; QIAO ET AL.: "Three-electrode method to study event-related responses in skin electrical potential, admittance and blood flow."**
• **MEDICAL AND BIOLOGICAL ENGINEERING AND COMPUTING. vol. 25, no. 6, November 1987, STEVENAGE GB pages 613 - 619; PRUNA ET AL.: "Dual-channel self-balancing electrodermal impedance reactometer for autonomic response studies,"**

**Description**

The invention refers to prognostic medicine, viz. to neurofysiology. The purpose of the invention is to increase the accuracy of the determination of the condition of the vegetative part of the nervous system, exposure or sympatheticotonia, parasympatheticotonia or normotonia (eutonia) by recording of the potentials from the skin of the body, or from the surface of the organs that can be reached by electrodes (stomach, mouth etc.), under the effect of the sensory or any other stimuli according to the amplitude or other indexes of the potentials.

The results of the measurements (electrovegetography-EVG) are estimated to record from fingers and toes from the palmary sides of the hands and feet or from other parts of the skin of the body, or from the surface of the organs that can be reached by electrodes, if possible symmetrically from the right and the left part from the body or many regions of the body. The active electrodes can be placed in the palmary side of the fingers and toes, right and left. The active electrodes can be placed also on any electro-active points of the skin or organs that can be reached. The passive electrodes can be placed on electrical low-active parts of the body.

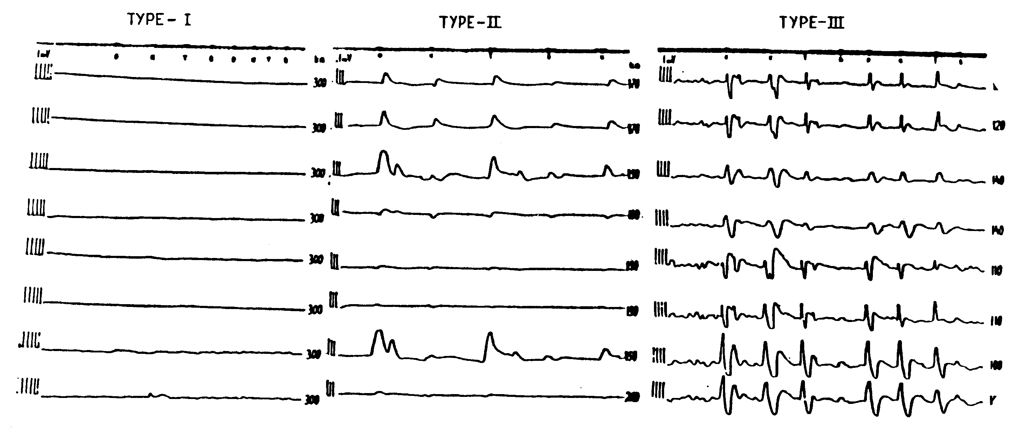

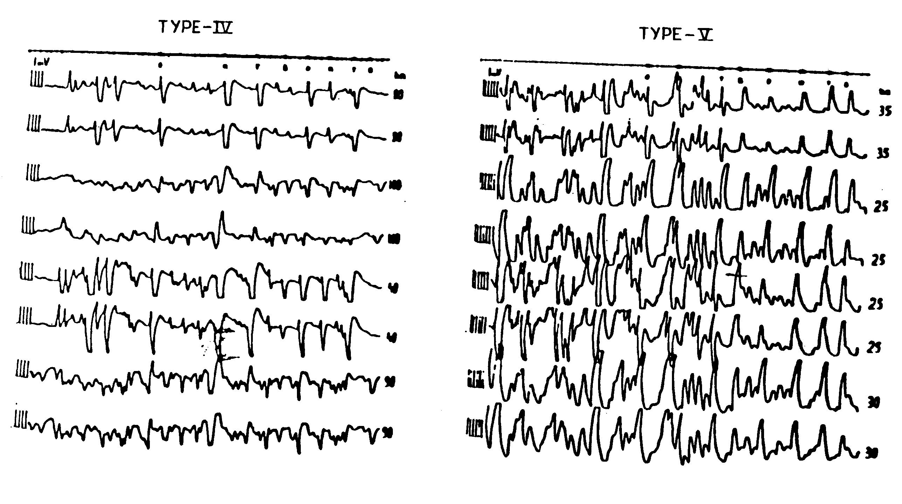

The registration of the potentials can be made from one point, two symmetrical points, from hands and feet at the same time or from combinations of some electro-active points of the skin or surface of organs that can be reached by electrodes, so the registration can be made polygraphical. During the registration stimulations (olfactory, electrical, visual, acustical, emotional etc.) are given five types of electrovegetograms (EVG) (Fig. 1) were found.

I. HYPERSYMPATHETICOTONIA (Fig. 1, type I): This type is characterized by the absence of background activity oscillations (up to 0.25 mV), stimuli do not evoke any responding reaction.

II. PRE-SYMPATHETICOTONIA (Fig. 1, type II): Background activity is almost absent as well (up to 0.25 mV) but responsive reaction to stimuli is distinctly revealed (0.5 mV and higher).

III. VEGETATIVE NORMOTONIA (EUTONIA) (Fig. 1, type III): Background activity is recorded, amplitude being from 0.25 to 1 mV. Responding reaction to stimuli is distinct (0.5 mV and higher).

IV. PREPARASYMPATHETICOTONIA (Fig. 1, type IV): Background activity is marked (1-2 mV), responsive reaction to stimuli is distinct (1 mV and higher).

V. HYPERPARASYMPATHETICOTONIA (Fig. 1, type V): Background activity is high (2-5 mV), responsive reaction to stimuli is distinct (1 mV and higher).

Spontaneous oscillations of the potential types III, IV and V are, as a rule, two-phase, sometimes single-phase (more often negative) deviations from an isoelectric line with frequency of 4-22 oscillations a minute. These oscillations are of different voltage, relatively rhythmic, sometimes in groups.

TABLE

| TYPES OF EVG'S | | | | |
|---|---|---|---|---|
| I | II | III | IV | V |
| 6.0 % | 31.5 % | 39.6 % | 18.8 % | 4 % |

The table shows the occurrence of the five types in normal persons . In 6 % of the cases the first type was found (hypersympatheticotonia), in 31.5 % - the second type (presympatheticotonia), in 39.6 % cases - type III (vegetative normotonia), in 18.8 % - type IV (preparasympatheticotonia) and in 4 % - type V (hyperparasympatheticotonia). Thus the extreme types I and V were found in 10 % of the patients.

In all the cases of EVG, studies were compared to a complex of clinical symptoms allowing to consider the predominance of the sympathetic or parasympathetic part of the vegetative nervous system. It is important to note that a number of signs give contradictory results. Therefore to judge the sympatheticotonia of vagotonia the following symptoms can be chosen, which symptoms are, as a rule, stable and most informative in estimating the condition of the vegetative nervous system:

heart rate, arterial pressure, respiratory rate, dermographism, extremities perspiration, clinoorthostatic tests, Aschner's test, infections tolerance, sleep, capability to work, salivation, absence or presence of dizziness, gastrointestinal disorders.

In all the patients and healty people studied, 95 % correlation between EVG-type and vegetative nervous system condition was found. Thus in all the persons studied, who have EVG type I, distinctly marked sympatheticotonia was observed, frequent-pulse of 80-100, white dermographism, dryness of palms and soles and most other signs of sympatheticotonia. In persons with EVG type II sympatheticotonia was also found (according to abovementioned symptoms) but less marked.

In persons with type III the condition of the vegetative part was balanced pulse rate 68-74 or the other way around, marked wetness or dryness of palms. In persons with EVG type IV signs of parasympatheticotonia were found (bradycardia, wettness of palms and soles, pink dermographism and other signs of vagotonia). In person with EVG type V these signs were distinctly marked.

These correlative data testify the informativeness of EVG as an express method allowing to diagnose the predominance of the sympathetic or parasympathetic part of the vegetative nervous system. EVG reveals among practically healthy people, those 10 % with severe pathology of the vegetative part of the nervous system, who are definitely threatened in future with severe diseases: hypertonic disease with insults and infarctions, glaucoma and other eye diseases, diseases of the gastro-intestinal tract (gastric ulcer, colitis, dyskinesias). In addition, EVG identifies another 50 % of the people who believe themselves healthy, but who also have significant alterations in vegetative innervation which is a most important "factor of risk", real probability of falling ill with one of the above-mentioned diseases. This refers to all the age groups of population, beginning with children of 6 to 7 and up to quite old people. Specific diseases and problems can sometimes be predicted using the method of the invention by placing the electrodes near or on the parts of the body where such disease or problem can occur, and by recording the potentials.

The registration of the potentials studied must be done by the apparatus with a time-constant from 1 until 5, preferably from 2 until 4 seconds. If an apparatus with a time constant of 3 seconds is used, then it is possible to registrate electrical waves from 3 or more waves/min.

$$\frac{1}{2\pi x 3sec} = \frac{1}{6.28x3sec} \approx \frac{1}{20sec} = 0.05 \text{ Hz} = 3/\text{min.}$$

It is possible to use an amplifier with other characteristics but then the data of sympatheticotonia and parasympatheticotonia will be different (another frequency, another amplitude character and form of the oscillations etc.)

It may be in some cases, that in some skin regions the potentials are much lower than on others in the immediate surroundings. This can give wrong indications. To avoid wrong findings it is preferred to search the skin region with a split electrode, of which the two electrodes come together by one registration. For example, the potential from fingers will be registrated by two electrodes. So one electrode is put on the phalange of the second finger and one on the phalange of the third finger. It might be necessary to put other electrodes on the phalanges of other fingers and all electrodes come together in one thread. If the skin of one finger is thick, the potential will be recorded from the other finger. In this way the risk of making no registration mistakes is minimized.

EXAMPLES

Case I

Patent B, aged 29, diagnosis: practically healthy, pulse 72/m. arterial pressure 120/75 mm Hg, respiration 12/min.

| Tests | clinostatic | | standing | 74 |
|---|---|---|---|---|
| | | | recumbent | 68 |
| | orthostatic | | recumbent | 68 |
| | | | standing | 76 |
| | Aschner's test | | initial | 62 |
| | | | afterwards | 54 |
| | sweating | hands | a little moist at usual temperature | |
| | | feet | a little moist at usual temperature | |
| | dermographism | | hands and feet pink. | |

It is difficult to determine the condition of the vegetative part of the nervous system, judging by clinical studies. Electrovegetogram (EVG) was recorded for this patient using the method described.

Potentials with the amplitude up to 1 mV and the frequency of 18/min were recorded; distinct reciprocal potentials (1 mV) to the stimulans of a light bulb of 25 Watt during the period of 1 second, were found, i.e. EVG suggests vegetative normotonia. In this condition the patient is practically healthy.

Case II

Patient C, aged 24, diagnosis: practically healthy. Pulse 70/min, arterial pressure 115/70 mm Hg, respiration 14/min.

| Tests | clinostatic | | standing | 72 |
|---|---|---|---|---|
| | | | recumbent | 68 |
| | orthostatic | | recumbent | 64 |
| | | | standing | 74 |
| | Aschner's test | | initial | 64 |
| | | | afterwards | 54 |
| | sweating | hands | a little moist at usual temperature | |
| | | feet | a little moist at usual temperature | |
| | dermographism | | hand and feet pink. | |

It is also difficult to determine the condition of the vegetative part of the nervous system. EVG was registrated for this patient. In the background curve, potentials with the amplitude up to 1 mV and frequency of 14/min. were recorded. Distinct reciprocal potentials (1 mV) to acoustical stimuli were found, that is EVG suggests vegetative normotonia.

Case III

Patient K, aged 25, diagnosis: practically healthy. Pulse 72, arterial pressure 120/70 mm Hg, respiration 14/min.

| Tests | clinostatic | | standing | 70 |
|---|---|---|---|---|
| | | | recumbent | 64 |
| | orthostatic | | recumbent | 62 |
| | | | standing | 70 |
| | Aschner's test | | initial | 64 |
| | | | afterwards | 56 |
| | sweating | hands | a little moist at usual temperature | |
| | | feet | a little moist at usual temperature | |
| | dermographism | | hand and feet pink. | |

It is difficult to determine the condition of the vegetative part of the nervous system. In the background curve of EVG, potential with amplitude up to 1 mV and frequency of 14/min were recorded.

Distinct reciprocal potentials (1 mV) to electrical stimulation of 10-15 mA were found, that is, EVG suggests vegetative normotonia.

Case IV

Patient O, aged 30. diagnosis: practically healthy. Pulse 70/min, arterial pressure 115/75 mm Hg, respiration 14/min.

| Test | clinostatic | | standing | 70 |
|---|---|---|---|---|
| | | | recumbent | 62 |
| | orthostatic | | recumbent | 64 |
| | | | standing | 72 |
| | Aschner's test | | initial | 62 |
| | | | afterwards | 56 |
| | sweating | hands | a little moist at usual temperature | |
| | | feet | a little moist at usual temperature | |
| | dermographism | | hands and feet pink. | |

In the background curve of the EVG, potentials with the amplitude up to 1 mV and frequency of 11/min. were recorded, distinct reciprocal potentials (1 mV) to olfactory stimuli (camphora, eau de Cologne) were found (Fig. 1, type III) that is EVG also suggests vegetative normotonia.

Case V

Patient L, aged 23, diagnosis: practically healthy, pulse 90/min., arterial pressure 140/100 mm Hg, respiration 22/min.

| Test | clinostatic | | standing | 92 |
|---|---|---|---|---|
| | | | recumbent | 86 |
| | orthostatic | | recumbent | 84 |
| | | | standing | 100 |
| | Aschner's test | | initial | 86 |
| | | | afterwards | 84 |
| | sweating | hands | dry, cool | |
| | | feet | dry, cool | |
| | dermographism | | hands and feet white. | |

Thus clinical studies of this patient suggest sympatheticotonia, but it is difficult to determine its degree. The EVG of the patient was recorded at rest and under the effect of light stimulations. The background activity is absent at all leads, that is, the potential do not exceed 0.25 mV, the light stimulations do not arouse any reaction at the EVG (Fig. 1, type I). The EVG data make it possible to draw a conclusion, that the patient has marked sympatheticotonia. The clinical signs and the character of the EVG show that it is hypersympatheticotonia. Based on this conclusion it was recommended that the patient should undergo treatment, decreasing the tonus of the sympathetic part of the vegetative nervous system (warm baths, diet poor of proteins etc.).

Case VI

Patient H, aged 28, diagnosis: practically healthy, pulse 86/min., arterial pressure 125/90 mm Hg, respiration 19/min.

| Tests | clinostatic | | standing | 86 |
|---|---|---|---|---|
| | | | recumbent | 82 |
| | orthostatic | | recumbent | 80 |
| | | | standing | 94 |
| | Aschner's test | | initial | 84 |
| | | | afterwards | 80 |
| | sweating | hands | dry, cool | |
| | | feet | dry, cool | |
| | dermographism | | hands white, feet a little pink. | |

Thus clinical studies of this patient suggest also sympatheticotonia, but it is difficult to determine its degree. The EVG of the patient shows that the background activity is absent (the potentials do not exceed 0.25 mV). Light stimulation causes distinct reciprocal potentials up to 1 mV. The clinical and EVG data make it possible to draw a conclusion, that the patient has sympatheticotonia, but not so strong as in Case V. This type of EVG belongs to preparasympatheticotonia (type IV).

Case VII

Patient K, aged 20, diagnosis: practically healthy, pulse 62/min. arterial pressure 100/60 mm Hg, respiration 11/min.

| Tests | clinostatic | | standing | 66 |
|---|---|---|---|---|
| | | | recumbent | 54 |
| | orthostatic | | recumbent | 54 |
| | | | standing | 60 |
| | Aschner's test | | initial | 62 |
| | | | afterwards | 50 |
| | sweating | hands | sweatly, cool | |
| | | feet | sweatly, cool | |
| | dermographism | | hands and feet red. | |

Clinical symptomatics allow to think of the prevalence of the parasympathetic part of the nervous system, but it is difficult to determine the degree of this prevalence. Using the method of the invention, an electrovegetogram of the patient was recorded. The background activity at a voltage of 1-2 mV and a frequency of 12-14 oscillations/min. was found. The reciprocal reactions of light stimuli were distinct (1-3 mV) which allows to draw a conclusion of distinct parasympatheticotonia (Fig. 1, type IV - preparasympatheticotonia). Based on this conclusion a treatment aimed at the decrease in the parasympathetic tonus is recommended.

Case VIII

Patient O, aged 26, diagnosis: practically healthy, pulse 54/min, arterial pressure 90/60 mm Hg, respiration 8/min.

| Tests | clinostatic | | standing | 58 |
|---|---|---|---|---|
| | | | recumbent | 44 |
| | orthostatic | | recumbent | 50 |
| | | | standing | 56 |
| | Aschner's test | | initial | 60 |
| | | | afterwards | 44 |
| | sweating | hands | very sweaty, cool | |
| | | feet | very sweaty, cool | |

Clinical symptoms also show that in this patient the parasympathetic part of the nervous system prevales, but it is difficult to determine the degree of this prevalence exactly. In the background activity of the EVG there are oscillations with a voltage of up to 5 mV and a frequency of 12-16/min. The reciprocal reaction of light stimuli was distinct (2-5 mV), which allows to draw a conclusion of distinctly marked parasympatheticotonia (Fig. 1, type V - hyperparasympatheticotonia).

Case IX

Patient S, aged 24, married, has a child. Diagnosis: practically healthy, pulse 56/min., arterial pressure 100/60 mm Hg, respiration 10/min.

| Tests | clinostatic | | standing | 58 |
|---|---|---|---|---|
| | | | recumbent | 50 |
| | orthostatic | | recumbent | 50 |
| | | | standing | 56 |
| | Aschner's test | | initial | 56 |
| | | | afterwards | 48 |
| | sweating | hands | sweaty | |
| | | feet | cool | |
| | dermographism | hands | red | |
| | | feet | stable. | |

The patient seems to have parasympatheticotonia. According to EVG data, type V is revealed, which confirms parasympatheticotonia. In this patient skin potentials from the upper third of the hips inner surface on both sides were recorded. High voltage potentials with amplitude of 6-8 mV and frequency of 8-12/min. evoke potentials of 5-8 mV. According to her answers to the interrogatory this woman is characterized by increased sexuality, which is confirmed by skin potentials nature on both femoral inner surfaces.

Case X

Patient K, aged 26, married, has a child. Pulse 60/min., arterial pressure 90/60 mm Hg, respiration 126/min.

| Tests | clinostatic | | standing | 64 |
|---|---|---|---|---|
| | | | recumbent | 50 |
| | orthostatic | | recumbent | 50 |
| | | | standing | 56 |
| | Aschner's test | | initial | 56 |
| | | | afterwards | 52 |
| | sweating | hands | sweaty | |
| | | feet | cool | |
| | dermographism | hands | red | |
| | | feet | stable. | |

According to clinical features and EVG data (type V), the patient has marked parasympatheticotonia. Recording to skin potentials from the upper third of the inner femoral surfaces in both sides shows practically absent potentials (up to - 0.25 mV).

Olfactori stimuli in EVG evoke responding potentials with amplitude up to 0.5-0.7 (EVG of type II). According to the patient's words, she is marked with hyposexuality, libido is absent.

Thus, EVG recording from the hips inner surfaces upper thirds marks it possible to diagnose the degree of patient's sexuality.

**Claims**

1. Method of determining the condition of the vegetative part of the nervous system of a person by recording the potentials from areas of the body of the person, wherein the person is given a sensory stimulus and both the background potential oscillations and the potential responses to the stimulus are recorded by means of electrodes placed on the skin or on the surface of an organ, and wherein the amplitudes of the oscillations and of the responses are used together to determine the condition of the nervous system.

2. Method according to claim 1, wherein the stimulus is given repeatedly.

3. Method according to claim 1 or 2, wherein:

   - the absence of background oscillation of up to 0.25 mV together with the absence of a potential response to the stimulus is identified as hypersympatheticotonia (type I);
   - the absence of background oscillations together with a response to the stimulus of 0.5 mV or higher is identified as presympatheticotonia (type II);
   - the presence of background oscillations from 0.25 to 1 mV together with a stimulus response of 0.5 mV or higher is identified as vegetative normotonia or eutonia (type III);
   - the presence of marked background oscillations from 1 to 2 mV together with a stimulus response of 0.5 mV or higher is identified as preparasympatheticotonia (type IV); and
   - the presence of high background oscillations together with a stimulus response of 0.5 or higher, in particular 1 mV and higher, is identified as parasympatheticotonia (type V).

4. Method according to claim 3, wherein the recording is repeated during two or more days, especially when one of types III, IV and V is identified.

5. Method according to any of claims 1-4, wherein the recording is performed on more than one part of the body.

**Patentansprüche**

1. Verfahren zur Bestimmung des Zustandes des vegetativen Teils des Nervensystems einer Person durch Aufzeichnung des Potentiale von Bereichen des Körpers der Person, bei dem der Person ein sensorischer Stimulus gegeben wird und sowohl das Hintergrundoszillationspotential als auch das auf dem Stimulus reagierende Potential mittels Elektroden, die auf der Haut oder der Oberfläche eines Organs angebracht sind, aufgezeichnet werden, und bei dem die Amplituden der Oszillationen und der Reaktionen gemeinsam zur Bestimmung des Zustandes des Nervensystems genutzt werden.

2. Verfahren gemäß Anspruch 1, bei dem der Stimulus wiederholt gegeben wird.

3. Verfahren nach Anspruch 1 oder 2, bei dem:

- die Abwesenheit von Hintergrundoszillation von bis zu 0,25 mV zusammen mit der Abwesenheit eines auf den Stimulus reagierenden Potentials als Hypersympathetikotonie (Typ I) identifiziert wird;

- die Abwesenheit von Hintergrundoszillationen zusammen mit einer Reaktion auf den Stimulus von 0,5 mV oder höher als Präsympathetikotonie (Typ II) identifiziert wird;

- die Anwesenheit von Hintergrundoszillationen von 0,25 bis 1 mV zusammen mit einer Stimulusreaktion von 0,5 mV oder höher als vegetative Normotonie oder Eutonia (Typ III) identifiziert wird;

- die Anwesenheit von ausgeprägten Hintergrundoszillationen von 1 bis 2 mV zusammen mit einer Stimulusreaktion von 0,5 mV oder höher als Präparasympathetikotonie (Typ IV) identifiziert wird; und

- die Anwesenheit von hohen Hintergrundoszillationen zusammen mit einer Stimulusreaktion von 0,5 oder höher, insbesondere 1 mV und höher, als Parasympathetikotonie (Typ V) identifiziert wird.

4. Verfahren nach Anspruch 3, bei dem die Aufzeichnung während zwei oder mehr Tage wiederholt wird, insbesondere wenn einer der Typen III, IV und V identifiziert wird.

5. Verfahren nach einem der Ansprüche 1-4, bei dem die Aufzeichnung an mehr als einem Körperteil durchgeführt wird.

**Revendications**

1. Procédé de détermination de l'état de la partie végétative du système nerveux d'une personne par enregistrement des potentiels des zones du corps de la personne, dans lequel un stimulus sensoriel est appliqué à la personne et à la fois les oscillations du potentiel de fond et les réponses de potentiel au stimulus sont enregistrées à l'aide d'électrodes placées sur la peau ou à la surface d'un organe, et dans lequel les amplitudes des oscillations et des réponses servent conjointement à déterminer l'état du système nerveux.

2. Procédé selon la revendication 1, dans lequel le stimulus est appliqué de manière répétée.

3. Procédé selon la revendication 1 ou 2, dans lequel :

- l'absence d'oscillations de fond de jusqu'à 0,25 mV, jointe à l'absence d'une réponse de potentiel au stimulus, est identifiée comme une hypersympathéticotonie (type I) ;
- l'absence d'oscillations de fond, jointe à une réponse au stimulus égale ou supérieure à 0,5 V, est identifiée comme une présympathéticotonie (type II) ;
- la présence d'oscillations de fond comprises entre 0,25 et 1mV, jointe à une réponse de stimulus égale ou supérieure à 0,5 mV, est identifiée comme une normotonie ou une eutonie végétative (type III);
- la présence d'oscillations de fond marquées, comprises entre 1 et 2 mV, jointe à une réponse au stimulus égale ou supérieure à 0,5 mV, est identifiée comme une préparasympathéticotonie (type IV) ; et
- la présence de fortes oscillations de fond, jointe à une réponse au stimulus égale ou supérieure à 0,5 mV, en particulier égale ou supérieure à 1mV, est identifiée comme une parasympathéticotonie (type V).

4. Procédé selon la revendication 3, dans lequel l'enregistrement est répété pendant deux jours ou plus, en particulier lorsqu'un des types III, IV et V est identifié.

5. Procédé selon l'une quelconque des revendications 1 à 4, dans lequel l'enregistrement est effectué sur plus d'une partie du corps.

TYPE—V

TYPE—IV